# EUROPÄISCHE PATENTSCHRIFT

(11) **EP 0 838 456 B1**
(45) Veröffentlichungstag und Bekanntmachung des Hinweises auf die Patenterteilung: **05.01.2000**
(21) Anmeldenummer: 97118719.0
(22) Anmeldetag: 28.10.1997
(51) Int. Cl.: C07D 211/58

(54) **Verfahren zur Herstellung von 4-Amino-2,2,6,6-tetramethylpiperidin (TAD) über das Zwischenprodukt 2,2,6,6-Tetramethyl-4-[(2,2,6,6-tetramethyl-4-piperidyliden)amino]-piperidin**
Process for preparation of 4-amino-2,2,6,6-tetramethylpiperidine (TAD) via the intermediate 2,2,6,6-tetramethyl-4-[(2,2,6,6-tetramethyl-4-piperidylidene)amino]-piperidine
Procédé pour la préparation de 4-amino-2,2,6,6-tétraméthylpipéridine (TAD) via le composé intermédiaire 2,2,6,6-tétraméthyl-4-[(2,2,6,6-tétraméthyl-4-pipéridylidéne)amino]-pipéridine

(30) Priorität: 28.10.1996 DE 19644770
(43) Veröffentlichungstag der Anmeldung: 29.04.1998
(73) Patentinhaber: BASF AKTIENGESELLSCHAFT, 67056 Ludwigshafen (DE)
(72) Erfinder: Julius, Manfred, Dr., 67117 Limburgerhof (DE)
(74) Vertreter: Isenbruck, Günter, Dr.

(56) Entgegenhaltungen:
- EP-A- 0 033 529
- EP-A- 0 336 895
- EP-A- 0 714 890
- DE-A- 3 525 387

## Beschreibung

Die Erfindung betrifft ein Verfahren zur Herstellung von 4-Amino-2,2,6,6-tetramethyl-piperidin (TAD) über das Zwischenprodukt 2,2,6,6-Tetramethyl-4-(2,2,6,6-tetramethyl-4-piperidyliden)amino]-piperidin.

4-Amino-2,2,6,6-tetramethyl-piperidin (Triacetondiamin, TAD) ist ein zentrales Zwischenprodukt für die Synthese von Lichtschutzmitteln vom HALS-Typ (HALS = Hindered Amine Light Stabilizer). Diese spielen als Additive für die Stabilisierung von synthetischen Polymeren, wie zum Beispiel Polyolefinen eine wichtige Rolle. Es ist bekannt, daß synthetische Polymere unter dem Einfluß von Sonnenlicht oder ultravioletter Strahlung aus anderen Quellen sich mehr oder weniger stark chemisch oder physikalisch ändern. Um diese nachteilige Wirkung der ultravioletten Strahlung auf synthetische Polymere zu verhindern bzw. zu verzögern, werden den Polymeren geeignete Lichtschutzmittel etwa vom vorstehend genannten HALS-Typ zugesetzt.

Aus der EP-A 0 033 529 ist ein Verfahren zur Herstellung von 4-Amino-2,2,6,6-tetramethyl-piperidin (TAD) bekannt, wobei man 2,2,6,6-Tetramethylpiperidinon-4 (TAA) in Gegenwart von Hydrierkatalysatoren mit Ammoniak und Wasserstoff bei Temperaturen von 120 bis 220°C und einem Druck in einem Bereich von 150 bis 500 bar behandelt. TAA reagiert jedoch empfindlich auf O₂ bzw. Luft, was zu farbgebenden Verunreinigungen führt. Diese finden sich nach Durchführung des erwähnten Verfahrens in den Produkten wieder oder müssen zur Herstellung der Lichtstabilisatoren abgetrennt werden.

Als Alternative zu dem bekannten Verfahren wird deshalb nunmehr ein Verfahren zur Herstellung von 4-Amino-2,2,6,6-tetramethyl-piperidin (TAD) der Formel I 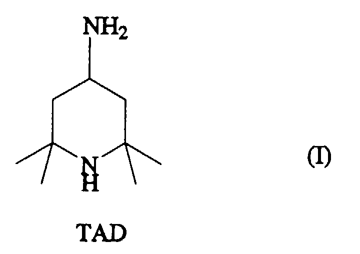 beschrieben, das die Schritte umfaßt, daß in einem ersten Schritt 2,2,6,6-Tetramethyl-piperidin-4-on (TAA) der Formel II 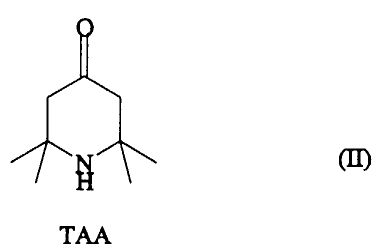 mit TAD zu 2,2,6,6-Tetramethyl-4-[(2,2,6,6-tetramethyl-4-piperidyliden) amino]-piperidin (Imin III) gemäß der folgenden Formel III 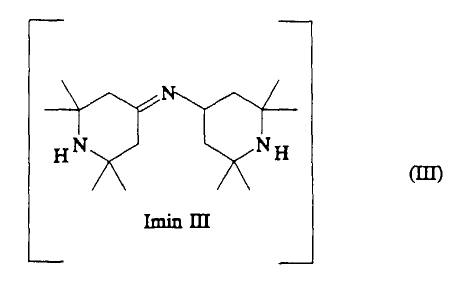 umgesetzt und in einem weiteren Schritt das Imin (III) in Gegenwart eines Hydrierkatalysators mit Ammoniak und Wasserstoff zu 2 Moläquivalenten TAD reagieren gelassen wird.

Außerdem stellt die Erfindung ein Verfahren zur Herstellung von 2,2,6,6-Tetramethyl-4-[(2,2,6,6-tetramethyl-4-piperidyliden)amino]-piperidin der vorstehend genannten Formel III bereit, wobei das Verfahren den Schritt umfaßt, daß 2,2,6,6-Tetramethyl-piperidin-4-on (TAA) der nachfolgenden Formel II 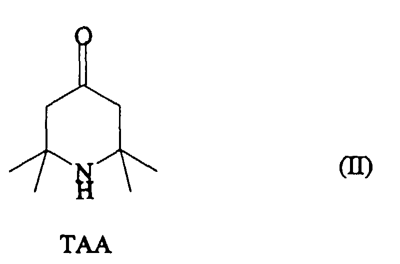 mit 4-Amino-2,2,6,6-tetramethyl-piperidin (TAD) der Formel I umgesetzt wird.

Gemäß einer weiteren Ausführungsform der Erfindung wird TAD mittels des vorstehend zuerst genannten Verfahrens unter Weglassen des Umsetzungsschritts von TAA mit TAD aus dem Imin III hergestellt, wobei das Imin III in Gegenwart eines Hydrierkatalysators mit Ammoniak und Wasserstoff zu 2 Moläquivalenten TAD reagieren gelassen wird.

Erfindungsgemäß wurde gefunden, daß sich TAA mit TAD unter Freisetzung von Wasser zu dem Imin (III) umsetzen läßt. Auf diese Weise läßt sich das Imin III mit hoher Selektivität und guter Ausbeute herstellen.

Zur Herstellung des TAD erfolgt anschließend eine aminierende Hydrierung an einem Hydrierkatalysator, ebenfalls mit sehr hoher Selektivität bei fast vollständigem Umsatz. Auf diese Weise werden bei dem erfindungsgemäßen Verfahren unter Einsatz je eines Moläquivalents TAA und TAD zwei Moläquivalente TAD erhalten.

Die verschiedenen erfindungsgemäßen Ausführungsformen des Verfahrens werden beispielhaft durch die nachfolgende Reaktionsgleichung beschrieben:

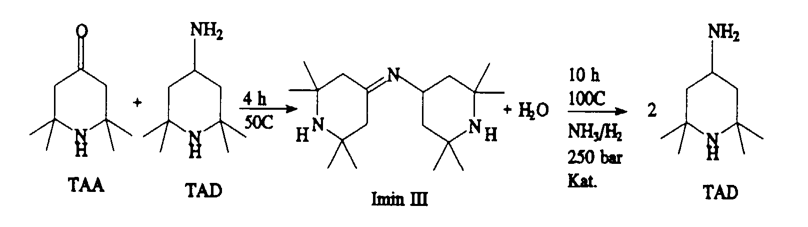

Bevorzugt erfolgt die vorstehend wiedergegebene Kondensation von TAA mit TAD zu dem Imin (III) nicht katalysiert bei Temperaturen im Bereich von 30 bis 90°C.

Ganz besonders vorteilhaft ist bei dem erfindungsgemäßen Verfahren, daß es in einer einstufigen Fahrweise durchgeführt werden kann. Dabei wird das als Zwischenprodukt auftretende Imin nicht als Reinsubstanz isoliert, sondern kann unter üblichen Bedingungen, beispielsweise bei 100°C, 250 bar H₂, NH₃-Überschuß, direkt weiter zu TAD aminierend hydriert werden.

Des weiteren wird das Verfahren bevorzugt ohne zusätzliches Lösungsmittel durchgeführt. Es kann außerdem sowohl diskontinuierlich im Autoklaven, z.B an einem handelsüblichen Suspensions- oder Festbettkatalysator, als auch kontinuierlich im Rohrreaktor, z.B. an einem Festbettkatalysator, durchgeführt werden.

Bevorzugte Hydrierkatalysatoren enthalten Ni und/oder Co.

Das erfindungsgemäße Verfahren wird durch die nachfolgenden Beispiele erläutert:

### Beispiel 1

### Synthese von 4-Amino-2,2,6,6-tetramethyl-piperidin (TAD)

In einem 1,2 l HC-Autoklaven mit Begasungsrührer wurden 85,95 g (0,55 Mol) TAD (Reinheit nach GC: 98,8 Fl.-%), 77,62 g (0,50 Mol) TAA (Reinheit nach GC: 99,8 Fl.-%) und 50 g Raney-Cobalt vorgelegt. Nach dem Inertisieren mit N₂ wurde auf 50°C erhitzt und 4 h bei Eigendruck gerührt. Die Reaktionsmischung besaß danach ca. folgende Zusammensetzung (Fl.-% nach GC): 15,8 % TAA, 8,5 % TAD, 72,5 % Imin III, 3,2 % Rest (GC-Bedingungen: Kapillarsäule 30 m RTX-5 Amine; 50-250°C, 5°C/Min.) Man kühlte den Autoklaveninhalt auf Raumtemperatur ab (wobei die Reaktionsmischung erstarrte), preßte 170 g (10,0 Mol) flüssigen Ammoniak auf und erhitzte auf 100°C. Unter Rühren wurde der Druck mit Wasserstoff auf 250 bar erhöht und bei stündlichem Nachpressen auf 250 bar 10 h bei 100°C gefahren. Insgesamt wurden ca. 15 bar H₂ aufgenommen.

Nach dem Abkühlen und Entspannen wurde der Reaktionsaustrag in 225 g Methanol gelöst und vom Katalysator abdekantiert. Nach dem Einengen bei 40°C und 5 mbar wurden 157 g flüssiges Rohprodukt mit folgender Zusammensetzung erhalten (Fl.-% nach GC):
95,7 % TAD
3,2 % Triacetonaminoalkohol (TAA-ol)
0,4 % Imin III
0,7 % Rest.

Der Umsatz an Imin III betrug 99,4 % bei einer Selektivität von 98,5 %. Die Ausbeute an TAD, bezogen auf eingesetztes TAA, betrug 65,3 g (0,42 Mol, 84 %).

### Beispiel 2:

### Synthese von 2,2,6,6-Tetramethyl-4-[(2,2,6,6-tetramethyl-4-piperidyliden)amino]-piperidin (Imin III)

In einem Rührkolben wurden unter N₂ 85,95 g (0,55 Mol) TAD und 77,62 g (0,50 Mol) TAA vorgelegt und 4 h auf 50°C erhitzt. Nach GC besaß die Reaktionsmischung danach folgende Zusammensetzung (Fl.-%): 15,76 % TAA, 8,47 % TAD, 72,46 % Imin III, 3,31 % Rest (GC-Bedingungen: Kapillarsäule 30 m RTX-5 Amine; 50-250°C/Min.).
Dies entspricht einer Selektivität für die Bildung des Imins von 95,6 % bei einem Umsatz von 75,8%.

Das reine, weiße Imin wurde durch Kristallisation aus Petrolether 30/75 gewonnen. Smp.: 59-61°C.

| Elementaranalyse: | | | | |
|---|---|---|---|---|
| | C | H | N | |
| % ber. | 73,66 | 12,02 | 14,32 | C₁₈H₃₅N₃ (MG=293,50) |
| % gef. | 73,3 | 12,1 | 14,2 | |

¹³C-NMR (62,9 MHz, CDCl₃):
δ=28,76 (2·CH₃), 31,45 (2·CH₃), 31,87 (2·CH₃), 35,06 (2·CH₃), 42,11 (CH₂-CH), 46,05 (2·CH₂-C=N), 50,51 (2·C(CH₃)₂), 51,40 (C(CH₃)₂), 52,26 (C(CH₃)₂), 53,72 (CH-N=C), 54,07 (CH₂-CH), 168,28 (C=N).
Massenspektrum (El): M⁺ = 293.
IR-Spektrum: 1650 cm⁻¹ (C=N).

## Patentansprüche

1. Verfahren zur Herstellung von 4-Amino-2,2,6,6-tetramethyl-piperidin (TAD) der Formel I 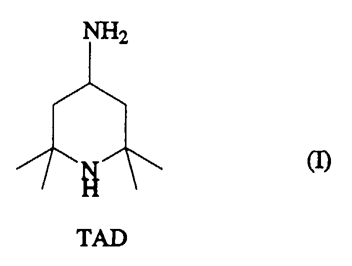 umfassend die Schritte, daß
- in einem ersten Schritt 2,2,6,6-Tetramethyl-piperidin-4-on (TAA) der Formel II 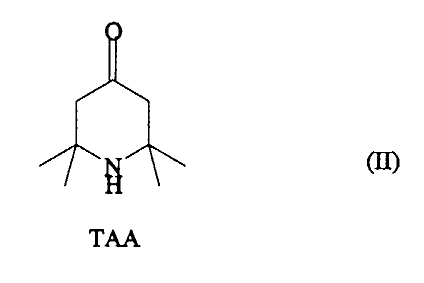 mit TAD zu 2,2,6,6-Tetramethyl-4-[(2,2,6,6-tetramethyl-4-piperidyliden)amino]-piperidin (Imin III) gemäß der folgenden Formel III 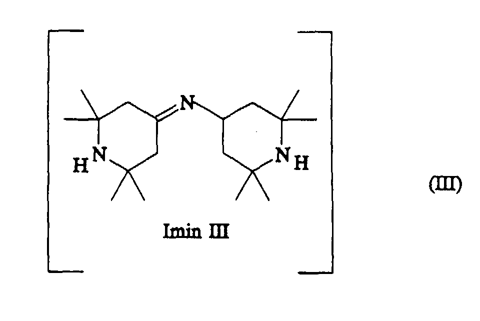 umgesetzt wird und
- in einem weiteren Schritt das Imin (III) in Gegenwart eines Hydrierkatalysators mit Ammoniak und Wasserstoff zu 2 Moläquivalenten TAD reagieren gelassen wird.

2. Verfahren nach Anspruch 1, wobei der weitere Verfahrensschritt des Reagierenlassens des Imins (III) in Gegenwart eines Hydrierkatalysators mit Ammoniak und Wasserstoff direkt im Anschluß an den ersten Verfahrensschritt erfolgt.

3. Verfahren nach einem der Ansprüche 1 oder 2, wobei der weitere Verfahrensschritt des Reagierenlassens des Imins (III) mit Ammoniak und Wasserstoff bei Temperaturen im Bereich von 20°C bis 150°C und einem Druck von 50 bis 300 bar lösungsmittelfrei erfolgt.

4. Verfahren nach einem der Ansprüche 1 bis 3, wobei das TAD unter Weglassen des ersten Verfahrensschritts aus dem Imin m hergestellt wird.

5. Verfahren zur Herstellung von 2,2,6,6-Tetramethyl-4-[(2,2,6,6-tetramethyl-4-piperidyliden)amino]-piperidin der nachfolgenden Formel III 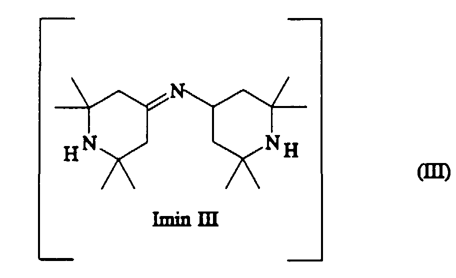 wobei das Verfahren den Schritt umfaßt, daß 2,2,6,6-Tetramethyl-piperidin-4-on (TAA) der nachfolgenden Formel II 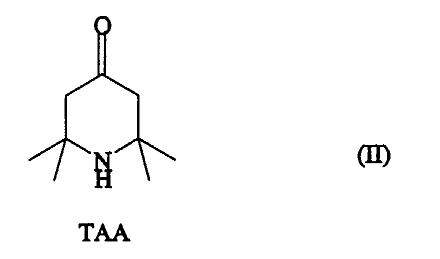 mit 4-Amino-2,2,6,6-tetramethyl-piperidin (TAD) der Formel I 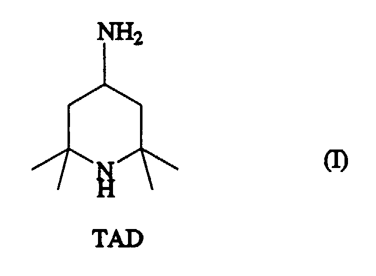 umgesetzt wird.

## Claims

1. A process for preparing 4-amino-2,2,6,6-tetramethylpiperidine (TAD) of the formula I 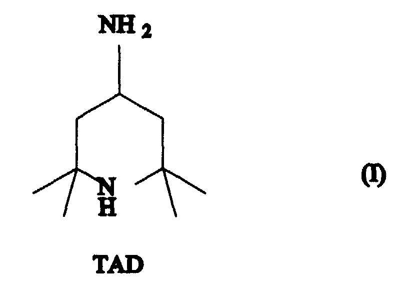 which comprises the steps of reacting
- in a first step, 2,2,6,6-tetramethylpiperidin-4-one(TAA) of the formula II 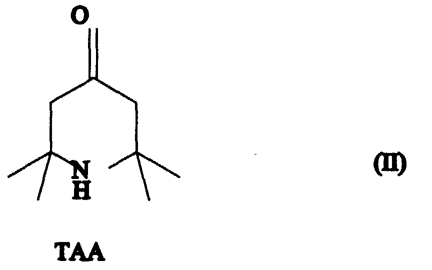 with TAD to form 2,2,6,6-tetramethyl-4-[(2,2,6,6-tetramethyl-4-piperidylidene)amino]piperidine (imine III) of the formula III below 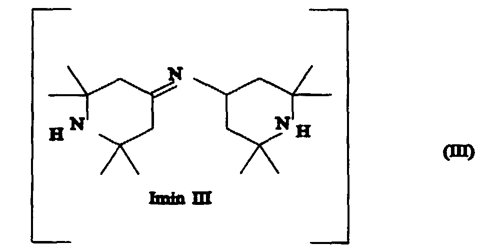 and of reacting
- in a further step, the imine (III) with ammonia and hydrogen in the presence of a hydrogenation catalyst to give 2 mol equivalents of TAD.

2. A process as claimed in claim 1, wherein the further process step of reacting the imine (III) with ammonia and hydrogen in the presence of a hydrogenation catalyst is carried out directly after the first process step.

3. A process as claimed in either of claims 1 and 2, wherein the further process step of reacting the imine (III) with ammonia and hydrogen is carried out at from 20 to 150°C and under a pressure of from 50 to 300 bar in the absence of solvents.

4. A process as claimed in any of claims 1 to 3, wherein the TAD is prepared from the imine III with omission of the first process step.

5. A process for preparing 2,2,6,6-tetramethyl-4-[(2,2,6,6-tetramethyl-4-piperidylidene)amino]piperidine of the formula III below 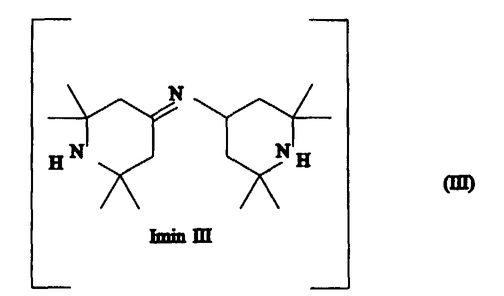 which comprises the step of reacting 2,2,6,6-tetramethylpiperidin-4-one (TAA) of the formula II below 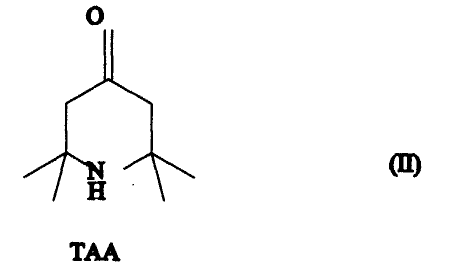 with 4-amino-2,2,6,6-tetramethylpiperidine (TAD) of the formula I

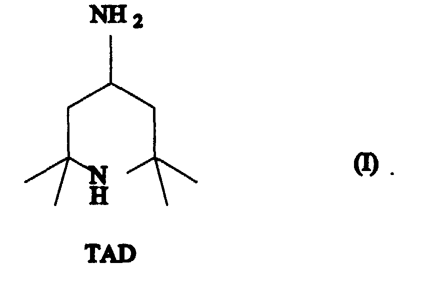

## Revendications

1. Procédé pour la préparation de 4-amino-2,2,6,6-tétraméthyl-pipéridine (TAD) de formule I 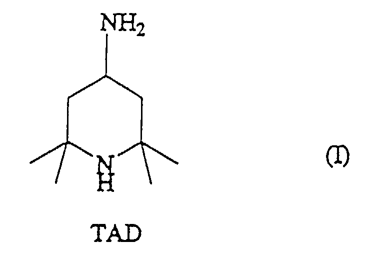 comprenant les étapes consistant à
- convertir dans une première étape la 2,2,6,6-tétraméthyl-pipéridine-4-one (TAA) de formule II 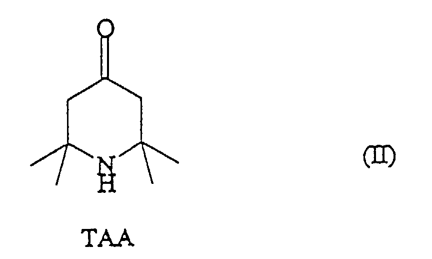 avec de la TAD pour former de la 2,2,6,6-tétraméthyl-4-[(2,2,6,6-tétraméthyl-4-pipéridylidène)amino]-pipéridine (imine III) correspondant à la formule III ci-après 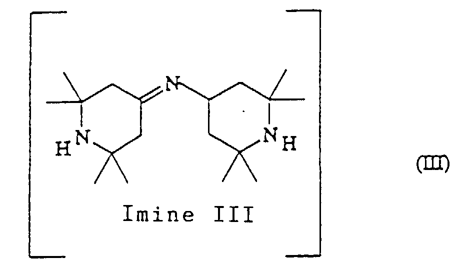 et
- faire réagir dans une autre étape l'imine (III) en présence d'un catalyseur d'hydrogénation avec de l'ammoniac et de l'hydrogène pour former 2 équivalents molaires de TAD.

2. Procédé selon la revendication 1, dans lequel l'autre étape opératoire consistant à faire réagir l'imine (III) en présence d'un catalyseur d'hydrogénation avec de l'ammoniac et de l'hydrogène est mise en oeuvre directement à la suite de la première étape opératoire.

3. Procédé selon l'une des revendications 1 ou 2, dans lequel l'autre étape opératoire consistant à faire réagir l'imine (III) avec de l'ammoniac et de l'hydrogène est effectuée à des températures comprises dans l'intervalle de 20°C à 150°C sous une pression de 50 à 300 bar.

4. Procédé selon l'une des revendications 1 à 3, dans lequel la TAD est préparée à partir de l'imine III en l'absence de la première étape opératoire.

5. Procédé pour la préparation de 2,2,6,6-tétraméthyl-4-[(2,2,6,6-tétraméthyl-4-pipéridylidène)amino]-pipéridine répondant à la formule III ci-après 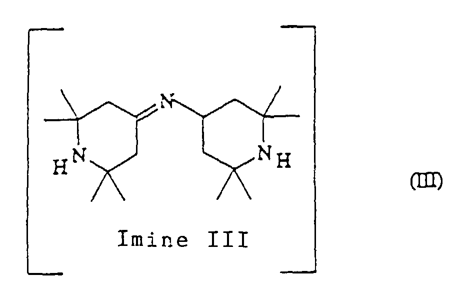 tandis que le procédé comprend l'étape consistant à faire réagir de la 2,2,6,6-tétraméthyl-pipéridine-4-one (TAA) de formule II ci-après 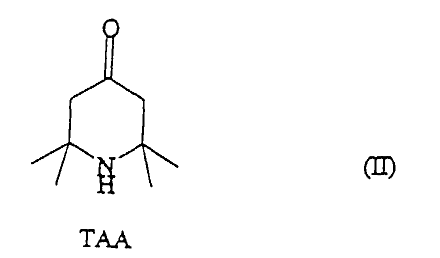 avec de la 4-amino-2,2,6,6-tétraméthyl-pipéridine (TAD) de formule I
